(19) Europäisches Patentamt / European Patent Office / Office européen des brevets

(11) **EP 1 521 601 B2**

(12) **NEUE EUROPÄISCHE PATENTSCHRIFT**

Nach dem Einspruchsverfahren

(45) Veröffentlichungstag und Bekanntmachung des Hinweises auf die Entscheidung über den Einspruch:
**02.08.2017 Patentblatt 2017/31**

(45) Hinweis auf die Patenterteilung:
**07.05.2008 Patentblatt 2008/19**

(21) Anmeldenummer: **03763772.5**

(22) Anmeldetag: **09.07.2003**

(51) Int Cl.:
**A61L 15/60** (2006.01)

(86) Internationale Anmeldenummer:
**PCT/EP2003/007425**

(87) Internationale Veröffentlichungsnummer:
**WO 2004/006971 (22.01.2004 Gazette 2004/04)**

(54) **WASSERABSORBIERENDE, SCHAUMF RMIGE POLYMERGEBILDE**

WATER-ABSORBING, FOAM-TYPE POLYMER STRUCTURES

STRUCTURES POLYMERES, SOUS FORME DE MOUSSES, HYDROABSORBANTES

(84) Benannte Vertragsstaaten:
**AT BE BG CH CY CZ DE DK EE ES FI FR GB GR HU IE IT LI LU MC NL PT RO SE SI SK TR**

(30) Priorität: **11.07.2002 DE 10231356**

(43) Veröffentlichungstag der Anmeldung:
**13.04.2005 Patentblatt 2005/15**

(73) Patentinhaber: **Evonik Degussa GmbH**
**45128 Essen (DE)**

(72) Erfinder:
• **HINTZ, Sandra**
**47443 Moers (DE)**
• **BRÜGGEMANN, Helmut**
**47447 Moers (DE)**

(74) Vertreter: **Herzog, Fiesser & Partner**
**Patentanwälte PartG mbB**
**Immermannstrasse 40**
**40210 Düsseldorf (DE)**

(56) Entgegenhaltungen:
| | |
|---|---|
| **EP-A- 0 293 762** | **EP-A- 0 347 241** |
| **EP-A1- 0 744 435** | **EP-A1- 0 802 238** |
| **WO-A-97/17397** | **WO-A1-00/52087** |
| **WO-A1-87/03208** | **WO-A2-03/026707** |
| **WO-B1-95/02002** | **US-A- 5 149 335** |
| **US-A- 5 260 345** | **US-A- 5 672 633** |

• **BUCHHOLZ F. ET AL: 'Modern Superabsorbent Polymer Technology', WILEY-VCH, NEW YORK Seiten 199 - 215**
• **Versuchsbericht = M. Linsenbühler u. F.J. López Villanueva**

**Beschreibung**

Die Erfindung betrifft ein Verfahren zur Herstellung eines wasserabsorbierenden, schaumförmigen Polymergebildes und ein Verfahren zur Herstellung eines Verbundes.

Wasserabsorbierende Polymere werden als Superabsorber oder superabsorbierende Polymere bezeichnet, da sie in der Lage sind, ein Vielfaches ihres Eigengewichtes an wässrigen Flüssigkeiten unter Ausbildung von Hydrogelen aufzunehmen. In der Praxis werden diese Superabsorber beispielsweise in Windeln zur Absorption von Urin eingesetzt. Sie haben die Eigenschaft, die absorbierte Flüssigkeit auch unter mechanischer Belastung zurückzuhalten.

Die Verwendung der Superabsorber geschieht heute vorwiegend in pulverförmiger Ausführung. Im Zuge von Verfahrensvereinfachungen besteht der Wunsch, die Superabsorber in einer fixierten Form einzusetzen, beispielsweise in eine geschäumte Matrix einzubinden. Gemäß dem Stand der Technik gibt es zwei unterschiedliche Typen von geschäumten Systemen.

Beim ersten Typ handelt es sich um eine geschäumte Matrix, welche vorgefertigtes Superabsorbergranulat enthält. So beschreibt EP-A-0 427 219 Mischungen aus superabsorbierenden Polymeren und Latexschäumen. Die geschäumte Matrix dient dabei zur Fixierung des Superabsorbergranulats und zur Flüssigkeitsverteilung. Die Matrix selbst trägt allerdings zur Flüssigkeitsabsorption nur sehr begrenzt bei.

Bei dem zweiten Typ handelt es sich um Schäume, die selber aus superabsorbierendem Material bestehen. Zur Herstellung derartiger Schäume werden in der Literatur drei unterschiedliche Verfahren beschrieben. Diese können entweder durch Polymerisation einer Monomerenlösung in einer Wasser-in-Öl-Polymerdispersion (i), durch Schäumen einer Monomerlösung und anschließende Polymerisation (ii) oder durch thermisches Schäumen eines Polymers unter Zusatz von Bläh- bzw. Treibmitteln (iii) hergestellt werden.

So beschreiben WO 96/21680 und WO 96/21681 ein nach einem Verfahren des Typs (i) hergestellten Schaum. Bei diesem Verfahren wird eine Monomermischung, die eine emulgierte wässrige Phase enthält, polymerisiert. Das Wasser wirkt hierbei als Platzhalter f-ur die späteren Poren des Schaums. Die in diesem Dokument beschriebenen Schäume weisen allerdings eine geringe Retention auf.

WO 94/22502 beschreibt ein nach einem Verfahren des Typs (ii) erhaltenen Schaum auf Basis teilneutralisierter Polyacrylate. Der Schaum wird durch Aufschäumen einer Monomermischung mit einem wasserlöslichen Treibmittel, wie Freon 1,1,2, hergestellt. Der Nachteil der in diesem Dokument beschriebenen Schäume liegt in ihrer geringen Absotptionsgeschwindigkeit.

WO 97/17397 werden absorbierende Schäume durch das Aufschäumen einer Monomerlösung mit Blähmitteln nach einem Verfahren des Typs (ii) erhalten. Der Nachteil der in diesem Dokument beschriebenen Schäume liegt in ihrer Härte und Brüchigkeit. Um sie verarbeiten zu können, müssen Weichmacher in die Schäume eingearbeitet werden.

US 4,394,930 beschreibt absorbierende Polymerschäume, die nach einem Verfahren des Typs (iii) hergestellt wurden. In dem in diesem Dokument beschriebenen Verfahren werden vorgefertigte Superabsorber, wie beispielsweise Stärke-Copolymere, unter Zusatz von Blähmitteln auf einer Unterlage wie etwa einem Zellstoffvlies oder einer Polyethylenfolie thermisch geschäumt. Die durch dieses Verfahren erhaltenen Schäume sind hart und wenig flexibel.

WO 88/09801 beschreibt ebenfalls absorbierende Polymerschäume, die nach einem Verfahren des Typs (iii) erhalten wurden. Ein Terpolymer aus Ethylacrylat, Na-Acrylat und Na-Methacrylat wird in Gegenwart von Treibmitteln, wie beispielsweise Natriumhydrogencarbonat, thermisch geschäumt. Aufgrund des geringen Polyelektrolytanteils verfügen die in diesem Dokument beschriebenen Schäume allerdings über eine geringe Retention.

Allgemein liegt die erfindungsgemäße Aufgabe darin, die sich aus dem Stand der Technik ergebenden Nachteile zu überwinden.

Eine weitere, dieser Erfindung zugrunde liegende Aufgabe besteht darin, absorbierende Polymerschäume bereitzustellen, die sowohl hinsichtlich der Retention als auch hinsichtlich der Absorptionsgeschwindigkeit und der Absorption unter einer Belastung zufriedenstellende Eigenschaften aufweisen.

Ferner liegt die erfindungsgemäße Aufgabe darin, absorbierende Schäume bereitzustellen, die ein hohe Weichheit und Flexibilität besitzen, obwohl sie aus einem einheitlichen Polymer bestehen, in die kein vorgefertigtes Superabsorbergranulat eingearbeitet werden muss.

Des Weiteren liegt dieser Erfindung die Aufgabe zugrunde, ein Verfahren bereitzustellen, mit dem derartige Schäume durch den Einsatz möglichst kostengünstiger Ausgangsverbindungen und möglichst ohne den Einsatz halogenierter Kohlenwasserstoffe als Treibmittel hergestellt werden können.

Schließlich besteht eine erfindungsgemäße Aufgabe darin, Verbunde, insbesondere Hygieneartikel und deren Bestandteile bereitzustellen, die neben den Saugeigenschaften einen hohen Tragekomfort aufweisen und insbesondere den Benutzer nicht in sehrer Beweglichkeit einschränken.

Die vorstehenden Aufgaben werden gelöst durch ein Verfahren zur Herstellung von wasserabsorbierenden, schaumförmigen Polymergebilden, wobei eine wässrige Zusammensetzung (A) beinhaltend

(A1) Wasser,

(A2) ein oder mehrere Polymere, welche mindestens auf

($\alpha$1) 55-100 Gew.%, vorzugsweise 55-99,9 Gew.% und besonders bevorzugt 70-90 Gew.% einem polymerisierten, monoethylenisch ungesättigten, säuregruppenhaltigen Monomeren oder dessen Salz, sowie auf
($\alpha$2) 0-45 Gew.%, vorzugsweise 0,1-45 Gew.% und besonders bevorzugt 10-30 Gew.-% einem polymerisierten, monoethylenisch ungesättigten, mit ($\alpha$1) copolymerisierbaren Monomeren basiert,
wobei die Summe der Gewichtsmengen ($\alpha$1) und ($\alpha$2) 100 Gew.% beträgt und wobei mindestens 31,5 Gew.-% der Monomeren, vorzugsweise mindestens 50 Gew.% und besonders bevorzugt mindestens 75 Gew.-%, jeweils bezogen auf das Gesamtgewicht der Monomeren ($\alpha$1) und ($\alpha$2), Acrylsäure oder Salze der Acrylsäure sind,

(A3) einen oder mehrere Vernetzer,
(A4) ein oder mehrere Blähmittel,
(A5) ein oder mehrere Tenside,
(A6) sowie gegebenenfalls weitere Hilfsstoffe

aufgeschäumt und die geschäumte, wässrige Zusammensetzung anschließend bei einer Temperatur in einem Bereich von 50 bis 300°C, vorzugsweise in einem Bereich von 100 bis 250°C erhitzt wird, so dass, vorzugsweise wodurch, die Polymere (A2) zumindest teilweise vernetzt und der Gehalt an Wasser (A1) auf höchstens 15 Gew.%, bevorzugt auf höchstens 10 Gew.-% und besonders bevorzugt auf höchstens 5 Gew.-%, jeweils bezogen auf das Gesamtgewicht des entstehenden, schaumförmigen Polymergebildes und jeweils bestimmt nach der Ofenmethode gemäß ERT 430.1-99, eingestellt wird.

In einer anderen Ausführungsform des erfindungsgemäßen Verfahrens kann das vorstehend erhaltenen schaumförmige Polymergebilde mindestens ein weiteres mal mit mindestens einem Vernetzer in Kontakt gebracht werden. Als Vernetzer sind die Vernetzer (A3) bevorzugt. Die weitere Vernetzung des schaumförmigen Polymergebildes erfolgt vorzugsweise thermisch, bevorzugt in einem Bereich von 50 bis 300°C und besonders bevorzugt in einem Bereich von 120 bis 200°C. Besonders bevorzugt erfolgt die Vernetzung stärker im Bereich der Oberfläche des schaumförmigen Polymergebildes.

In einer bevorzugten Ausführungsform des erfindungsgemäßen Verfahrens sind mindestens 50 Mol%, bevorzugt mindestens 90 Mol% und besonders bevorzugt mindestens 99,9 Mol% der Monomere des Polymers (A2) wasserlöslich.

Die monoethylenisch ungesättigten, säuregruppenhaltigen Monomere ($\alpha$1) können teilweise oder vollständig, bevorzugt teilweise neutralisiert sein. Vorzugsweise sind die monoethylenisch ungesättigten, säuregruppenhaltigen Monomere zu mindestens 25 Mol%, besonders bevorzugt zu mindestens 50 Mol% und darüber hinaus bevorzugt zu 50-90 Mol% neutralisiert. Die Neutralisation der Monomere ($\alpha$1) kann vor oder auch nach der Polymerisation erfolgen. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak oder mit Alkalimetallhydroxiden, besonders bevorzugt mit Natriumhydroxid oder mit Ammoniak.

Bevorzugte monoethylenisch ungesättigte, säuregruppenhaltige Monomere ($\alpha$1) sind Acrylsäure, Methacrylsäure, Ethacrylsäure, $\alpha$-Chloracrylsäure, $\alpha$-Cyanoacrylsäure, $\beta$-Methylacrylsäure (Crotonsäure), $\alpha$-Phenylacrylsäure, $\beta$-Acryloxypropionsäure, Sorbinsäure, $\alpha$-Chlorsorbinsäure, 2'-Methylisocrotonsäure, Zimtsäure, p-Chlorzimtsäure, $\beta$-Stearylsäure, Itaconsäure, Citraconsäure, Mesaconsäure, Glutaconsäure, Aconitsäure, Maleinsäure, Fumarsäure, Tricarboxyethylen und Maleinsäureanhydrid, wobei Acrylsäure sowie Methacrylsäure besonders und Acrylsäure darüber hinaus bevorzugt sind.

Neben diesen carboxylatgruppenhaltigen Monomeren sind als monoethylenisch ungesättigte, säuregruppenhaltige Monomere ($\alpha$1) des Weiteren ethylenisch ungesättigte Sulfonsäuremonomere oder ethylenisch ungesättigte Phosphonsäuremonomere bevorzugt.

Ethylenisch ungesättigte Sulfonsäuremonomere sind Allylsulfonsäure oder aliphatische oder aromatische Vinylsulfonsäuren oder acrylische oder methacrylische Sulfonsäuren bevorzugt. Als aliphatische oder aromatische Vinylsulfonsäuren sind Vinylsulfonsäure, 4-Vinylbenzylsulfonsäure, Vinyltoluolsulfonsäure und Stryrolsulfonsäure bevorzugt. Als Acryl- bzw. Methacrylsulfonsäuren sind Sulfoethyl(meth)acrylat, Sulfopropyl(meth)acrylat, 2-Hydroxy-3-methacryloxypropylsulfonsäure und 2-Acrylamido-2-methylpropansulfonsäure bevorzugt.

Ferner sind ethylenisch ungesättigte Phosphonsäuremonomere, wie Vinylphosphonsäure, Allylphosphonsäure, Vinylbenzylphosponsäure, (Meth)acrylamidoalkylphosphonsäuren, Acrylamidoalkyldiphosphonsäuren, phosponomethylierte Vinylamine und (Meth)acrylphosphonsäurederivate bevorzugt.

Bevorzugte monoethylenisch ungesättigte, mit ($\alpha$1) copolymerisierbare Monomere ($\alpha$2) sind Amide und Nitrile von monoethylenisch ungesättigten Carbonsäuren wie beispielsweise Acrylamid, Methacrylamid und N-Vinylformamide, Acrylonitril und Methacrylonitril, Dialkyldiallylammoniumhalide wie Dimethyldiallylammoniumchlorid, Diethyldiallylammoniumchlorid, Allylpiperidiniumbromid, N-Vinylimidazole wie N-Vinylimidazol, 1-Vinyl-2-methylimidazol und N-Vinylimidazoline wie N-Vinylimidazolin, 1-Vinyl-2-methylimidazolin, 1-Vinyl-2-ethylimidazolin oder 1-Vinyl-2-propylimidazolin, die in Form der freien Basen, in quarternisierter Form oder als Salz bei der Polymerisation eingesetzt werden können. Außerdem eignen sich Dialkylaminoalkylacrylate und Dialkylaminoalkylmethacrylate, zum Beispiel Dimethylaminoethylacrylat, Dimethylaminoethylmethacrylat, Diethylaminoethylacrylat und Diethylaminoethylmethacrylat. Andere bevorzugte Monomere ($\alpha$2) sind beispielsweise Vinylester von gesättigten $C_1$-$C_4$-Carbonsäuren wie beispielsweise Vinylformiat, Vinylacetat oder Vinylpropionat, Alkylvinylether mit mindestens zwei Kohlenstoffatomen in der Alkylgruppe, wie beispielsweise Ethylvinylether oder Butylvinylether, Ester monoethylenisch ungesättigter $C_3$-$C_6$-Carbonsäuren wie beispielsweise Ester aus einwertigen $C_1$-$C_8$-Alkoholen und Acrylsäure, Methacrylsäure oder Maleinsäure, Halbester von Maleinsäure, zum Beispiel Monomethylmaleat, und Hydroxyalkylester der genannten monoethylenisch ungesättigten Carbonsäuren, zum Beispiel 2-Hydroxyethylacrylat, Hydroxypropylacrylat, Hydroxybutylacrylat, Hydroxyethylmethacrylat, Hydroxypropylmethacrylat und Hydroxybutylmethacrylat, N-Vinyllactame wie beispielsweise N-Vinylpyrrolidon oder N-Vinylcaprolaetam, Acrylsäure und Methacrylsäureester von alkoxylierten, einwertigen, gesättigten Alkoholen, zum Beispiel von Alkoholen mit 10 bis 25 Kohlenstoffatomen, die mit 2 bis 200 Mol Ethylenoxid und/oder Propylenoxid pro Mol Alkohol umgesetzt worden sind, sowie Monoacrylsäureester von Polyethylenglykol oder Polypropylenglykol, wobei die Molmassen der Polyalkylenglykole beispielweise bis zu 2.000 betragen können. Weiterhin geeignete Monomere ($\alpha$2) sind alkylsubstituierte Styrole wie Ethylstyrol oder tert. Butylstyrol.

Die Herstellung der Polymere (A2) kann nach verschiedenen an sich bekannten Polymerisationsverfahren erfolgen. Bevorzugt ist die radikalische Polymerisation in homogener Phase, insbesondere in wässriger Lösung als sogenannte Gelpolymerisation. Weitere Möglichkeiten sind die Fällungspolymerisation aus organischen Lösungsmitteln, wie zum Beispiel aus Alkoholen, oder die Suspensions-, Emulsions- oder Mikroemulsionspolymerisation. In Sonderfällen sind anstelle der radikalischen Polymerisation auch über einen ionischen Mechanismus ablaufende Polymerisationen brauchbar. Bei der Polymerisation können neben den Polymerisationsinitiatoren weitere Adjuvantien, wie beispielsweise Kettenregler wie Mercaptoethanol, eingesetzt werden.

Als Initiatoren zur Initiierung der Polymerisation können alle unter den Polymerisationsbedingungen Radikale bildenden Initiatoren verwendet werden, die üblicherweise bei der Herstellung von Superabsorbern eingesetzt werden. Auch eine Initiierung der Polymerisation durch Einwirkung von Elektronenstrahlen auf die polymerisierbare, wässrige Monomerenmischung ist möglich. Die Polymerisation kann allerdings auch in Abwesenheit von Initiatoren der oben genannten Art durch Einwirkung energiereicher Strahlung in Gegenwart von Photoinitiatoren ausgelöst werden.

Polymerisationsinitiatoren sind vorzugsweise Peroxide, Hydroperoxide, Wasserstoffperoxid, Persulfate, Azoverbindungen sowie die sogenannten Redoxkatalysatoren. Bevorzugt ist der Einsatz wasserlöslicher Katalysatoren. In manchen Fällen ist es vorteilhaft, Mischungen verschiedener Polymerisationsinitiatoren zu verwenden. Unter diesen Mischungen sind die aus Wasserstoffperoxid und Natrium- oder Kaliumperoxodisulfat bevorzugt, die in jedem denkbaren Mengenverhältnis eingesetzt werden können. Geeignete organische Peroxide sind vorzugsweise Acetylacetonperoxid, Methylethylketonperoxid, t-Butylhydroperoxid, Cumolhydroperoxid, t-Amylperpivat, t-Butylperviat, t-Butylperneohexonat, t-Butylisobutyrat, t-Butylper-2-ethylhexenoat, t-Butylperisononanoat, t-Butylpermaleat, t-Butylperbenzoat, t-Butyl-3,5,5-tri-methylhexanoat und Amylperneodekanoat. Weiterhin sind als Polymerisationsinitiatoren bevorzugt: AzoVerbindungen, wie 2,2'-Azobis-(2-amidinopropan)dihydrochlorid, Azo-bis-amidinopropan-dihydrochlord, 2,2'-Azobis-(N,N-dimethylen)isobutyramidindihydrochlorid, 2-(Carbamoylazo)isobutyronitril und 4,4'-Azobis-(4-cyanovaleriansäure). Die genannten Verbindungen werden in üblichen Mengen eingesetzt, vorzugsweise in einem Bereich von 0,01 bis 5, bevorzugt von 0,1 bis 2 Mol-%, jeweils bezogen auf die Menge der zu polymerisierenden Monomere.

Die Redoxkatalysatoren enthalten als oxidische Komponente mindestens eine der oben angegebenen Perverbindungen und als reduzierende Komponente vorzugsweise Ascorbinsäue, Glukose, Sorbose, Manose, Ammonium- oder Alkalimetallhydrogensulfit, -sulfat, -thiosulfat, -hyposulfit oder sulfid, Metallsalze, wie Eisen-II-ionen oder Silberionen oder Natriumhydroxymethylsulfoxylat. Vorzugsweise wird als reduzierende Komponente des Redoxkatalysators Ascorbinsäure oder Natriumpyrosulfit verwendet. Bezogen auf die bei der Polymerisation eingesetzte Menge an Monomeren wird $1*10^{-5}$ bis 1 Mol-% der reduzierenden Komponente des Redoxkatalysators und $1*10^{-5}$ bis 5 Mol-% der oxidierenden Komponente des Redoxkatalysators eingesetzt. Anstelle der oxidierenden Komponente des Redoxkatalysators, oder in Ergänzung zu diesem, können ein oder mehrere, vorzugsweise wasserlösliche, Azoverbindungen verwendet werden.

Bevorzugt wird zur Herstellung der Polymere (A2) ein Redoxsystem bestehend aus Wassersoffperoxid, Natriumperoxodisulfat und Ascorbinsäure eingesetzt. Allgemein sind erfindungsgemäß Azoverbindungen als Initiatoren bevorzugt, wobei Azo-bis-amidinopropan-dihydrochlorid besonders bevorzugt ist. In der Regel wird die Polymerisation mit den Initiatoren in einem Temperaturbereich von 30 bis 90°C initiiert.

Wenn man die Polymerisation durch Einwirkung energiereicher Strahlung auslöst, verwendet man üblicherweise als Initiatoren sogenannte Photoinitiatoren. Hierbei kann es sich beispielsweise um sogenannte $\alpha$-Spalter, H-abstrahierende Systeme oder auch um Azide handeln. Beispiele für solche Initiatoren sind Benzophenon-Derivate wie Michlers-Keton, Phenanthren-Derivate, Fluoren-Derivate, Anthrachinon-Derivate, Thioxanthon-Derivate, Cumarin-Derivate, Benzoinether und deren Derivate, Azoverbindungen wie die oben genannten Radikalbildner, substituierte Hexaarylbisimidazole oder Acylphosphinoxide. Beispiele für Azide sind: (N,N-Dimethylamino)ethyl-4-azidocinnamat, 2-(N,N-Dimethylamino)ethyl-4-azidonaphthylketon, 2-(N,N-Dimethylamino)eihyl 4-azidobenzoat, 5-Azido-1-naphthyl-2-(N,N-dimethylamino)ethylsulfon, N-(4-Sulfonylazidophenyl)maleimid, N-Acetyl-4-sulfonylazidoanilin, 4-Sulfonylazidoanilin, 4-Azidoanilin, 4-Azidophenacylbromid, p-Azidobenzoesäure, 2,6-Bis(p-azidobenzyliden)cyclohexanon und 2,6-Bis(p-azidobenzyliden)-4-methylcyclohexanon.

In einer bevorzugten Ausführungsform des erfindungsgemäßen Verfahrens weisen die Polymere (A2) ein mittels GPC bestimmtes Zahlenmittel des Molekulargewichtes von mindestens von 10.000 g/mol, bevorzugt von mindestens 25.000 g/mol und darüber hinaus bevorzugt von mindestens 50.000 g/mol auf, wobei vorzugsweise ein mittels GPC bestimmtes Zahlenmittel des Molekulargewichtes von 10.000.000 g/mol, besonders bevorzugt von 5.000.000 g/mol nicht überschritten wird.

In einer weiteren bevorzugten Ausführungsform des erfindungsgemäßen Verfahrens wird vor dem Aufschäumen der wässrigen Zusammensetzung (A) in dieser ein Gehalt an Polymer (A2) von vorzugsweise 10 bis 90 Gew.-%, besonders bevorzugt von 20 bis 70 Gew.% und darüber hinaus bevorzugt von 30 bis 60 Gew.-%, jeweils bezogen auf das Gesamtgewicht der wässrigen Zusammensetzung (A), eingestellt. Vorzugsweise erfolgt dieses Einstellen durch Verdünnen der wässrigen Zusammensetzung (A) mit Wasser.

Erfindungsgemäß bevorzugte Vernetzer (A3) sind Verbindungen, die mindestens zwei funktionelle Gruppen aufweisen, die mit funktionellen Gruppen der Mono-, meren ($\alpha$1) oder ($\alpha$2) in einer Kondensationsreaktion (=Kondensadonsvernetzer), in einer Additionsreaktion oder in einer Ringöffnungsreaktion reagieren können (Vernetzerklasse I), oder polyvalente Metallkationen (Vernetzerklasse II). Dabei wird durch die Verbindungen der Vernetzerklasse I eine Vernetzung der Polymere durch Kondensationsreaktion der funktionellen Gruppen erreicht, während bei den Verbindungen der Vernetzerklasse II eine Vernetzung durch elektrostatische Wechselwirkung des polyvalenten Metallkations mit den funktionellen Gruppen der Monomere ($\alpha$1) oder ($\alpha$2) erreicht wird.

Als Verbindung der Vernetzerklasse I seien als Beispiele genannt Polyole, beispielsweise Ethylenglykol, Polethylenglykole wie Diethylenglykol, Triethylenglykol und Tetraethylenglykol, Propylenglykol, Polypropylenglykole wie Dipropylenglykol, Tripropylenglykol oder Tetrapropylenglykol, 1,3-Butandiol, 1,4-Butandiol, 1,5-Pentandiol, 2,4-Pentandiol, 1,6-Hexandiol, 2,5-Hexandiol, Glycerin, Polyglycerin, Trimethylolpropan, Polyoxypropylen, Oxyethylen-Oxypropylen-Blockcopolymere, Sorbitanfettsäureester, Polyoxyethylensorbitanfettsäureester, Pentaerythrit, Polyvinylalkohol und Sorbitol, Aminoalkohole, beispielsweise Ethanolamin, Diethanolamin, Triethanolamin oder Propanolamin, Polyaminverbindungen, beispielsweise Ethylendiamin, Diethylentriaamin, Triethylentetraamin, Tetraethylenpentaamin oder Pentaethylenhexaamin, Polyglycidylether-Verbindungen wie Ethylenglykoldiglycidylether, Polyethylenglykoldiglycidylether, Glycerindiglycidylether, Glycerinpolyglycidylether, Pentareritritpolyglycidylether, Propylenglykoldiglycidylether Polypropylenglykoldiglycidylether, Neopentylglykoldiglycidylether, Hexandiolglycidylether, Trimethylolpropanpolyglycidylether, Sorbitolpolyglycidylether, Phtahlsäurediglicidylester, Adipinsäurediglycidylether, 1,4-Phenylen-bis(2-oxazolin), Glycidol, Polyisocyanate, vorzugsweise Diisocyanate wie 2,4-Toluoldüsocyanat und Hexamethylendüsocyanat, Polyaziridin-Verbindungen wie 2,2-Bishydroxymethylbutanol-tris[3-(1-aziridinyl)propionat], 1,6-Hexamethylendiethylenharnstoff und Diphenylmethan-bis-4,4'-N,N'-diethylenharnstoff, Halogenepoxide beispielsweise Epichlor- und Epibromhydrin und $\alpha$-Methylepichlorhydrin, Alkylencarbonate wie 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on, 1,3-Dioxolan-2-on, Poly-1,3-dioxolan-2-on, polyquartäre Amine wie Kondensationsprodukte von Dimethylaminen und Epichlorhydrin. Als Verbindungen der Vernetzerklasse I sind des weiteren Polyoxazoline wie 1,2-Ethylenbisoxazolin, Vernetzer mit Silangruppen wie $\gamma$-Glycidoxypropyltrimethoxysilan und $\gamma$-Aminopropyltrimethoxysilan, Oxazolidinone wie 2-Oxazolidinon, Bis- und Poly-2-oxazolidinone und Diglykolsilikate bevorzugt.

Besonders bevorzugt als Vernetzer der Vernetzerklasse I ist Ethylencarbonat.

Die polyvalenten Metallkationen der Vernetzerklasse 11 leiten sich vorzugsweise von ein- oder mehrwertigen Kationen ab, die einwertigen insbesondere von Alkalimetallen, wie Kalium, Natrium, Lithium, wobei Lithium bevorzugt wird. Bevorzugte zweiwertige Kationen leiten sich von Zink, Beryllium, Erdalkalimetallen, wie Magnesium, Calcium, Strontium ab, wobei Magnesium bevorzugt wird. Weiter erfindungsgemäß einsetzbare höherwertige Kationen sind Kationen von Aluminium, Eisen, Chrom, Mangan, Titan, Zirkonium und andere Übergangsmetalle sowie Doppelsalze solcher Kationen oder Mischungen der genannten Salze. Bevorzugt werden Aluminiumsalze und Alaune und deren unterschiedliche Hydrate wie z. B. $AlCl_3 \times 6H_2O$, $NaAl(SO_4)_2 \times 12H_2O$, $KAl(SO_4)_2 \times 12H_2O$ oder $Al_2(SO_4)_3 \times 14\text{-}18H_2O$ eingesetzt.

Besonders bevorzugt werden $Al_2(SO_4)_3$ und seine Hydrate als Vernetzer der Vernetzungsklasse II verwendet.

Bevorzugte Ausführungsformen des erfindungsgemäßen Verfahrens ist ein Verfahren, bei dem Vernetzer der folgenden Vernetzerklassen bzw. der folgenden Kombinationen von Vernetzerklassen eingesetzt werden: I, II, I II.

Es ist erfindungsgemäß weiterhin bevorzugt, dass die Vernetzer (A3) in einer Menge in einem Bereich von 0,001 bis 10 Gew.-%, bevorzugt in einem Bereich von 0,01 bis 5 Gew.% und besonders bevorzugt in einem Bereich von 1 bis 2,5 Gew.%, jeweils bezogen auf das Gesamtgewicht der wässrigen Zusammensetzung (A) eingesetzt werden.

Als Blähmittel (A4) werden in dem erfindungsgemäßen Verfahren vorzugsweise Verbindungen eingesetzt, die unter den Verfahrensbedingungen zur Gasentwicklung fähig sind. Bevorzugte Blähmittel sind beispielsweise anorganische Salze, wie beispielsweise Ammoniumcarbonat oder Azodicarbonat, oder organische Verbindungen, die unter den Verfahrensbedingungen zur Decarboxylierung fähig sind, wie beispielsweise Citronensäure.

Es ist erfindungsgemäß weiterhin bevorzugt, dass die Blähmittel (A4) in einer Menge in einem Bereich von 0,1 bis 20 Gew.%, bevorzugt in einem Bereich von 1 bis 15 Gew.% und besonders bevorzugt in einem Bereich von 5 bis 12 Gew.-%, jeweils bezogen auf das Gesamtgewicht der wässrigen Zusammensetzung (A) eingesetzt werden.

Als Tenside (A5) können anionische, kationische, nichtionische oder ambivalente Tenside oder deren Mischungen verwendet werden. Es können sowohl niedermolekulare als auch polymere Tenside eingesetzt werden. Nichtionische Tenside sind beispielsweise Additionsprodukte von Alkylenoxiden, insbesondere Ethylenoxid, Propylenoxid und /oder Butylenoxid an Alkohole, Amine, Phenole, Naphthole oder Carbonsäuren. Vorteilhaft setzt man als Tenside Additionsprodukte von Ethylenoxid und/oder Propylenoxid an mindestens 10 Kohlenstoffatome enthaltende Alkohole ein, wobei die Additionsprodukte pro Mol Alkohol 3 bis 200 Mol Ethylenoxid und/oder Propylenoxid angelagert enthalten. Die Additionsprodukte enthalten die Alkylenoxid-Einheiten in Form von Blöcken oder in statistischer Verteilung. Beispiele für nichtionische Tenside sind die Additionsprodukte von 7 Mol Ethylenoxid an 1 Mol Talgfettalkohol, Umsetzungsprodukte von 9 Mol Ethylenoxid mit 1 Mol Talgfettalkohol und Additonsprodukte von 80 Mol Ethylenoxid mit 1 Mol Talgfettalkohol. Weitere handelsübliche nichtionische Tenside bestehen aus Umsetzungsprodukten von Oxoalkoholen oder Ziegler-Alkoholen mit 5 bis 12 Mol Ethylenoxid pro Mol Alkohol, insbesondere mit 7 Mol Ethylenoxid. Weitere handelsübliche nichtionische Tenside werden durch Ethoxylierung von Rizinusöl erhalten. Pro Mol Rizinusöl werden beispielsweise 12 bis 80 Mol Ethylenoxid angelagert. Weitere handelsübliche Produkte sind beispielsweise die Umsetzungsprodukte von 18 Mol Ethylenoxid mit 1 Mol Talgfettalkohol, die Additionsprodukte von 10 Mol Ethylenoxid an 1 Mol eines $C_{13}/C_{15}$ Oxoalkohols, oder die Umsetzungsprodukte von 7 bis 8 Mol Ethylenoxid an 1 Mol eines $C_{13}C_{15}$-Oxoalkohels. Weitere geeignete nichtionische Tenside sind Phenolalkoxylate wie beispielsweise p-tert.-Butylphenol, das mit 9 Mol Ethylenoxid umgesetzt ist, oder Methylether von Umsetzungsprodukten aus 1 Mol eines $C_{12}$-$C_{15}$-Alkohols und 7,5 Mol Ethylenoxid.

Die oben beschriebenen Tenside können beispielsweise durch Veresterung mit Schwefelsäure in die entsprechenden Schwefelsäurehalbester überführt werden. Die Schwefelsäurehalbester werden in Form der Alkalimetall- oder Ammoniumsalze als anionische Tenside eingesetzt. Als anionische Tenside eignen sich beispielsweise Alkalimetall- oder Ammoniumsalze von Schefelsäurehalbestem von Additionsprodukten von Ethylenoxid und/oder Propylenoxid an Fettalkohole, Alkalimetall- oder Ammoniumsalze von Alkylbenzolsulfonsäure oder Alkylphenolethersukfaten. Produkte der genannten Art sind im Handel erhältlich.

Auch kationische Tenside sind geeignet. Beispiele hierfür sind die mit Dimethylsufat quarternierten Umsetzungsprodukte von 6,5 Mol Ethylenoxid mit 1 Mol Oleylamin, Distearyldimetyhlammoniumchlorid, Lauryltrimethylammoniumchlorid, Cetylpyridiniumbromid und mit Dimethylsulfat quarternierter Stearinsäuretriethanolaminester.

Die Tenside (A5) sind in der wässrigen Zusammensetzung (A) vorzugsweise in einer Menge in einem Bereich von 0,01 bis 15 Gew.%, besonders bevorzugt in einem Bereich von 0,05 bis 10 Gew.-% und darüber hinaus bevorzugt in einem Bereich von 0,1 bis 5 Gew.-%, jeweils bezogen auf das Gewicht der wässrigen Zusammensetzung (A), enthalten.

Als Hilfsstoffe (A6) können in dem erfindungsgemäßen Verfahren Stabilisatoren, Verdicker, Füllstoffe oder Zellkeimbildner oder Mischungen hiervon eingesetzt werden.

Verdicker werden beispielsweise zur Optimierung der Schaumstruktur und zur Verbesserung der Schaumstabilität eingesetzt. Man erreicht damit, dass der Schaum während der Vernetzung der Polymere (A2) nur geringfügig schrumpft. Als Verdickungsmittel kommen alle hierfür bekannten natürlichen und synthetischen Polymere in Betracht, die die Viskosität eines wässrigen Systems stark erhöhen. Hierbei kann es sich um wasserquellbare oder wasserlösliche synthetische oder natürliche Polymere handeln. Als Verdicker sind auch pulverförmige Superabsorber geeignet. Eine ausführliche Übersicht über Verdicker findet man beispielsweise in den Veröffentlichungen von R. Y. Lochhead und W. R. Fron, Cosmetics & Toiletries, 108, 95-135 (Mai 1993) und M. T. Clarke, "Rheological Additivs" in D. Laba (ed.) "Rheological Properties of Cosmetics and Toiletries", Cosmetic Science and Technology Series, Volume 13, Marcel Dekker Inc., New York 1993, die einen Teil der Offenbarung bilden.

Als Füllstoffe werden vorzugsweise Kreiden, Betonite, Talkum, Kieselgele oder Kieselsäure, Aktivkohlen, Pigmente, wie Titandioxid und Eisenoxid oder deren Mischungen eingesetzt.

Die Hilfsstoffe (A6) sind in der wässrigen Zusammensetzung (A) vorzugsweise in einer Menge in einem Bereich von 0,01 bis 15 Gew.-%, besonders bevorzugt in einem Bereich von 0,05 bis 10 Gew.-% und darüber hinaus bevorzugt in einem Bereich von 0,1 bis 5 Gew.-%, jeweils bezogen auf das Gewicht der wässrigen Zusammensetzung (A), enthalten.

In einer bevorzugten Ausführungsform des erfindungsgemäßen Verfahrens wird die wässrige Zusammensetzung (A) dadurch erhalten, dass zu der nach der Polymerisation der Monomeren ($\alpha$1) und ($\alpha$2) in einer wässrigen Lösung erhaltenen, vorzugsweise wässrigen Polymerlösung die Vernetzer, die Blähmittel sowie gegebenenfalls die weiteren Hilfsstoffe zugesetzt werden.

Es ist erfindungsgemäß weiterhin bevorzugt, dass die wässrige Zusammensetzung (A) eine Viskosität gemäß ASTM-D 1824/90 bei 20 °C von mindestens 100 mPa·s, bevorzugt in einem Bereich von 100 bis 500.000 mPa·s und besonders bevorzugt in einem Bereich von 500 bis 5.000 mPa·s aufweist.

In einer bevorzugten Ausführungsform des erfindungsgemäßen Verfahrens wird die Zusammensetzung (A) derart aufgeschäumt, dass ein Schaumlitergewicht in einem Bereich von 10 bis 1.000 g/l, bevorzugt in einem Bereich von 50 bis 500 g/l und besonders bevorzugt in einem Bereich von 80 bis 250 g/l erhalten wird.

Das Aufschäumen der wässrigen Zusammensetzung erfolgt vorzugsweise durch mechanische Einwirkungen, insbesondere Scherung, besonders bevorzugt durch starkes Rühren oder Mixen unter Vermischung mit Luft. Es ist jedoch erfindungsgemäß auch möglich, die Zusammensetzung durch das Dispergieren eines inerten Gases in Form von feinen Gasblasen aufzuschäumen. Das Eintragen von Gasblasen in die wässrige Zusammensetzung (A) erfolgt beispielsweise mit Hilfe von Schlag-, Schüttel-, Rühr- oder Peitschvorrichtungen. Ferner ist es auch möglich, die Zusammensetzung dadurch aufzuschäumen, dass Gase aus einer flüssigkeitsbedeckten Öffnung ausströmen oder durch das Ausnutzen von Turbulenzerscheinungen in Strömungen. Des Weiteren kann auch die Ausbildung von Lamellen an Drähten oder Sieben für diesen Zweck genutzt werden. Diese unterschiedlichen Methoden können auch gegebenenfalls miteinander kombiniert werden. Als inerte Gase eignen sich beispielsweise Stickstoff, Kohlendioxid, Helium, Neon und Argon.

Das Erhitzen der aufgeschäumten Zusammensetzung erfolgt vorzugsweise in einem Ofen, einem Trockenschrank, mit einem heißen Gasstrom, durch Infrarotbestrahlung oder durch Mikrowellennestrahlung.

In einer bevorzugten Ausführungsform des erfindungsgemäßen Verfahren wird die geschäumte Zusammensetzung, bevor sie erhitzt wird, zunächst in einen Formkörper überführt. In einer weiteren bevorzugten AusMhrungsform wird die wässrige Zusammensetzung vor dem Aufschäumen in einen Formkörper überführt und anschließend in diesem Formkörper aufgeschäumt. Durch anschließendes Erhitzen der geschäumten Zusammensetzung in diesem Formkörper können dann wasserabsorbierende, schaumförmige Polymergebilde mit definierter Raumstruktur erhalten werden.

In einer weiteren, bevorzugten Ausführungsform des erfindungsgemäßen Verfahrens wird die Oberfläche des absorbierenden, schaumförmigen Polymergebildes in einem weiteren Verfahrensschritt geglättet, vorzugsweise mindestens teilsweise geglättet. Dabei erfolgt das Glätten der Oberfläche vorzugsweise durch ein Befeuchten der Oberfläche mittels Wasserdampf bei Temperaturen in einem Bereich von 20 bis 40°C und anschließendes Kalandern. Durch das Befeuchten und anschließende Kalandern werden die Poren im Oberflächenbereich des schaumrormigen Polymergebildes vorzugsweise lediglich geringfügig komprimiert, nicht jedoch gänzlich verschlossen.

Die Erfindung betrifft des weiteren ein wasserabsorbierendes, schaumförmiges Polymergebilde, welches durch das vorstehend beschriebenen Verfahren erhältlich ist.

In einer bevorzugten Ausführungsform des durch das erfindungsgemäße Verfahren erhältlichen wasserabsorbierenden, schaumförmigen Polymergebildes weist dieses mindestens eine der folgenden Eigenschaften auf:

($\beta$1) einen gemäß der hierin beschriebenen Testmethode bestimmte AUL *(Absorbency under Load)* von 0,9%er NaCl-Lösung bei einer Belastung von 0,3 psi von mindestens 10 g/g, bevorzugt von mindestens 13 g/g und besonders bevorzugt von mindestens 16 g/g;

($\beta$2) eine gemäß der hierin beschriebenen Testmethode bestimmte Absorptionsgeschwindigkeit von mehr als 1 g/g/sec, vorzugsweise mehr als 2 g/g/sec und besonders bevorzugt mehr als 3 g/g/sec;

($\beta$3) eine gemäß der hierin beschriebenen Testmethode bestimmte maximale Absorptionskapazität in einem Bereich von 20 bis 300 g/g, bevorzugt in einem Bereich von 30 bis 200 g/g und besonders bevorzugt in einem Bereich von 35 bis 100 g/g;

($\beta$4) eine gemäß der hierin beschriebenen Testmethode bestimmte CRC *(Centrifugation Retention Capacity)* in einem Bereich von 7,5 bis 100 g/g, bevorzugt in einem Bereich von 10 bis 80 g/g und besonders bevorzugt in einem Bereich von 15 bis 60 g/g;

($\beta$5) eine gemäß der hierin beschriebenen Testmethode bestimmte mittlere Porengröße in einem Bereich von 0,01 bis 2 mm, bevorzugt in einem Bereich von 0,1 bis 1 mm und besonders bevorzugt in einem Bereich von 0,2 bis 0,5 mm;

($\beta$6) ein gemäß der ERT 40.3-90 Testmethode bestimmtes mittleres Flächengewicht in einem Bereich von 60 bis 1200 g/m$^2$, bevorzugt in einem Bereich von 80 bis 800 g/m$^2$ und besonders bevorzugt in einem Bereich von 85 bis 500 g/cm$^2$.

Im Fall der Verwendung des schaumförmigen Polymergebildes in Damenhygienartikeln, insbesondere Damenbinden, ist ein Flächengewicht im Bereich von 60 bis 200 g/m$^2$ bevorzugt und im Bereich von 80 bis 1 00g/m$^2$ besonders bevorzugt.

Im Fall der Verwendung des schaumförmigen Polymergebildes in Bavbhygienartikeln, insbesondere Windeln, ist ein Flächengewicht im Bereich von 400 bis 1200 g/m$^2$ bevorzugt und im Bereich von 550 bis 800g/m$^2$ besonders bevorzugt.

Die sich aus den vorstehenden Eigenschaften ergebenden Eigenschaftskombinationen von zwei oder mehr dieser Eigenschaften stellen jeweils bevorzugte Ausführungsformen des erfindungsgemässen Polymergebildes dar.

Weiterhin als erfindungsgemässe Ausführungsförmen besonders bevorzugt sind Polymergebilde, welche die nachfolgend als Buchstaben oder Buchstabenkombinationen dargestellten Eigenschaften oder Eigenschaftskombinationen aufweisen: $\beta1$, $\beta2$, $\beta3$, $\beta4$, $\beta5$, $\beta6$, $\beta1\beta2$, $\beta1\beta3$, $\beta1\beta4$, $\beta1\beta5$, $\beta1\beta6$, $\beta2\beta3$, $\beta2\beta4$, $\beta2\beta5$, $\beta2\beta6$, $\beta3\beta4$, $\beta3\beta5$, $\beta3\beta6$, $\beta4\beta5$, $\beta4\beta6$, $\beta5\beta6$, $\beta1\beta2\beta3$, $\beta1\beta2\beta3\beta4$, $\beta1\beta2\beta3\beta4\beta5$, $\beta1\beta2\beta3\beta4\beta5\beta6$.

In einer bevorzugten Ausführungsform der durch das erfindungsgemäße Verfahren erhältlichen wasserabsorbierenden, schaumförmigen Polymergebilde weisen diese eine offenzellige Struktur, ein geschlossenzellige Struktur oder eine Mischstruktur aus offenen und geschlossenen Zellen, vorzugsweise jedoch eine offenzellige Struktur auf. Unter einer offenzelligen Struktur wird eine Struktur verstanden, bei der zwischen benachbarten Poren des Schaumes ein Flüssigkeitsaustausch möglich ist, während bei einer geschlossenzelligen Struktur die einzelnen Poren voneinander isoliert sind. Bei einer Mischstruktur aus offenzelligen und geschlossenzelligen Poren ist zwischen einigen benachbarten Poren ein Flüssigkeitsaustausch über gemeinsame Öffnungen mindestens zweier, benachbarter Poren möglich, während andere voneinander isoliert sind, so dass zwischen diesen kein Flüssigkeitsaustausch über gemeinsam Öffnungen in den Poren erfolgt. In einer bevorzugten Ausführungsform der durch das erfindungsgemäße Verfahren erhältlichen schaumförmigen Polymergebilde befinden sich mindestens 25%, bevorzugt mindestens 50% und darüber hinaus bevorzugt mindestens 75% der Poren in einem Flüssigkeitsaustausch mit mindestens einer benachbarten Pore.

Da die im Zusammenhang mit dem erfindungsgemäßen Verfahren beschriebene, geschäumte Zusammensetzung eine lange Standzeit aufweist, kann diese beispielsweise auf ein Substrat aufgebracht werden. Die Erfindung betrifft auch ein Verfahren zur Herstellung eines Verbund umfassend das wasserabsorbierende, schaumförmige Polymergebilde sowie ein Substrat, wobei die im Zusammenhang mit dem erfindungsgemäßen Verfahren beschriebene geschäumte Zusammensetzung mit mindestens einem Teil der Oberfläche eines Substrates in Kontakt gebracht wird und das mit der geschäumten Zusammensetzung in Kontakt gebrachte Substrat anschließend bei einer Temperatur in einem Bereich von 50 bis 300°C, bevorzugt eine einem Bereich von 100 bis 250°C erhitzt wird, so dass, bevorzugt wodurch, die Polymere (A2) zumindest teilweise vernetzt, der Gehalt an Wasser (A1) auf höchstens 15 Gew.-%, bevorzugt auf höchstens 10 Gew.-% und besonders bevorzugt auf höchstens 5 Gew.-%, jeweils bezogen auf das Gesamtgewicht des entstehenden, schaumförmigen Polymergebildes und jeweils bestimmt nach der Ofenmethode gemäß ERT 430.1-99, eingestellt und das entstehende, schaumförmige Polymergebilde auf zumindest einem Teil der Substratoberfläche immobilisiert wird (1. Verfahren).

In einer bevorzugten Ausfürungsform dieses erfindungsgemäßen Verfahrens erfolgt das in Kontakt bringen der geschäumten Zusammensetzung mit der Substratoberfläche durch das Aufbringen der Zusammensetzung, vorzugsweise durch Streichen, Rakeln oder Gießen, auf die Substratoberfläche in einer 0,1 bis 10 mm, bevorzugt in einer 1 bis 8 mm und besonders bevorzugt in einer 2 bis 4 mm dicken Schicht. Es ist erfindungsgemäß weiterhin bevorzugt, das die geschäumte Zusammensetzung in definierten Arealen, beispielsweise durch die Verwendung von Schablonen oder Sieben, auf dem Substrat aufgebracht wird. In einer weiteren bevorzugten Ausführungsform dieses Verfahrens wird die geschäumte Zusammensetzung auf Fluff-Schichten aufgetragen und die Fluff-Schichten somit mit der geschäumten Zusammensetzung imprägniert, so dass der Fluff nach dem Vernetzen integraler Bestandteil des Schaumes ist. Als Substrate im Zusammenhang mit diesem Verfahren sind Folien aus Polymeren, wie beispielsweise aus Polyethylen, Polypropylen oder Polyamid, Metalle, Vliese, Fluff, Tissues, Gewebe, natürliche oder synthetische Fasern, oder andere Schäume bevorzugt.

Ein Verbund beinhaltend das wasserabsorbierende, schaumförmige Polymergebilde kann auch dadurch erhalten werden, dass mindestens ein Teil der Oberfläche des wasserabsorbierenden, schaumförmigen Polymergebildes mit mindestens einem Teil der Oberfläche eines Substrates in Kontakt gebracht und das Polymergebilde anschließend auf mindestens einem Teil der Substratoberfläche immobilisiert wird (2. Verfahren). Das wasserabsorbierende, schaumförmige Polymergebilde kann dabei in Form von Polymergebilden mit definierter Raumstruktur, wie sie im Zusammenhang mit dem erfindungsgemäßen Verfahren zur Herstellung eines wasserabsorbierenden, schaumförmigen Polymergebildes beschrieben wurden, eingesetzt werden. Es ist jedoch auch möglich, Polymergebilde mit definierter Raumstruktur aus größeren Blöcken des Polymergebildes auszuschneiden oder auszusägen.

In einer bevorzugten Ausführungsform dieses Verfahrens erfolgt das Immobilisieren des Polymergebildes auf der Substratoberfläche durch mindestens einen der folgenden Verfahrensschritte:

($\delta$1) Aneinanderdrücken des Substrates und des wasserabsorbierenden, schaumförmigen Polymergebildes mit einem spezifischen Druck von mindestens 0,1 N/cm, bevorzugt von mindestens 0,5 N/cm und besonders bevorzugt von mindestens 1 N/cm oder Aneinanderdrücken des Substrates und des schaumförmigen Polymergebildes mit einem Druck von mindestens 0,1 N/cm$^2$, bevorzugt von mindestens 0,5 N/cm$^2$ und besonders bevorzugt von mindestens 1 N/cm$^2$;

($\delta$2) Erhitzen des mit dem schaumförmigen Polymergebilde in Kontakt gebrachten Substrates auf eine Temperatur in einem Bereich von 50 bis 300°C, bevorzugt eine einem Bereich von 100 bis 250°C.

Bevorzugte Ausführungsformen dieses Verfahrens sind Verfahren, die durch die folgenden Verfahrensschritte bzw. Kombinationen von Verfahrensschritten gekennzeichnet sind $\delta 1$, $\delta 2$, $\delta 1\delta 2$. Die Bedingungen der Verfahrensschritte $\delta 1$, $\delta 2$ werden vorzugsweise so gewählt, dass die Schaumstruktur nicht zerstört wird oder sich nach der Behandlung gemäß des vorstehend beschriebenen Verfahrens wieder bilden kann.

In einer bevorzugten Ausführungsform dieses Verfahrens erfolgt das Immobilisieren des wasserabsorbierenden, schaumförmigen Polymergebildes auf der Substratoberfläche durch Kalandern oder durch Bügeln des mit dem Polymergebilde in Kontakt gebrachten Substrates. Vorzugsweise wird dabei eine Schutzschicht auf die Seite des Substrates, die sich nicht in Kontakt mit dem Polymergebilde befindet, gelegt. Als Substrate im Zusammenhang mit diesem Verfahren sind thermoplastische Flächengebilde, vorzugsweise zumindest teilsweise schmelzbare Flächengebilde, wie beispielsweise Polyethylenfolien, bevorzugt.

Es ist erfindungsgemäß weiterhin bevorzugt, dass durch das erfindungsgemäße Verfahren zur Herstellung eines Verbundes sandwichartige Strukturen enthaltend mindestens zwei Schichten des wasserabsorbierenden, schaumförmigen Polymergebildes und mindestens zwei Substratschichten erhältlich sind, indem man in einem ersten Verfahrensschritt einen aus dem wasserabsorbierenden, schaumförmigen Polymergbilde (P1) und einem Substrat (S1) bestehenden Verbund entweder nach dem 1. oder 2. Verfahren herstellt, in einem zweiten Verfahrensschritt eine Schicht der geschäumten Zusammensetzung mit derjenigen Oberfläche des Substrates (S1), die der Kontaktfläche zwischen dem Polymergebilde (P1) und dem Substrat (S1) gegenüberliegt, in Kontakt bringt und, wie im 1. Verfahren beschrieben, unter Bildung eines wasserabsorbierenden, schaumformigen Polymergebildes (P2) immobilisiert und in einem dritten Verfahrensschritt diejenige Oberfläche des wasserabsorbierenden, schaumförmigen Polymergebildes (P2), die der Kontaktfläche zwischen dem Polymergebilde (P2) und dem Substrat (S1) gegenüberliegt, in Kontakt bringt und, wie im 2. Verfahren beschrieben, auf dem Substrat (S2) immobilisiert. Diese Verfahrensschritte können, je nach Anzahl der gewünschten Schichten, beliebig oft wiederholt werden.

Offenbart werden auch die durch das erfindungsgemäße Verfahren zur Herstellung eines Verbundes erhaltenen Verbunde. Hierunter sind die Fasern, insbesondere Fluff enthaltenden Verbunde sandwichartigen Strukturen bevorzugt, die als Core in Hygieneartikel eingesetzt werden können.

Offenbart wird die Verwendung der wasserabsorbierenden, schaumformigen Polymergebilde oder der Verbunde in chemischen Produkten. Bei den chemischen Produkten handelt es sich vorzugsweise um Fasern, Folien, Filme, Kabel, Dichtungsmaterialien, flüssigkeitsaufnehmende Hygieneartikel, Wundabdeckungen, Träger für pflanzen- und pilzwachstumsregulierende Mittel, Zusätze für Baustoffe, Verpackungsmaterialien und Bodenzusätze.

auch chemische Produkte beinhaltend die wasserabsorbierenden, schaumförmigen Polymergebilde oder die Verbunde. Bevorzugte chemische Produkte sind Fasern, Folien, Filme, Kabel, Dichtungsmaterialien, flüssigkeitsaufnehmende Hygieneartikel, Wundabdeckungen, Träger für pflanzen- und pilzwachstumsregulierende Mittel, Zusätze für Baustoffe, Verpackungsmaterialien und Bodenzusätze.

Unter den vorstehend genannten chemischen Produkten sind Hygieneartikel bevorzugt. Hierunter sind wiederum Damenbinden, Babywindeln, Erwachsenen Inkontinentzartikel, worunter Windeln besonders bevorzugt sind.

In einer besonders bevorzugten Ausführungsform ist der Verbund eine Windel. Hierbei stellen die Bestandteile der Windel, die von dem absorbierenden, schaumförmigen Polymergebilde verschieden sind, das Substrat des Verbundes dar. In einer bevorzugten Ausführungsform enthält die Windel ein zuvor beschriebenes Core. In diesem Fall stellen die von dem Core unterschiedlichen Bestandteile der Windel das Substrat des Verbundes dar. Im allgemeinen umfasst ein als Windel eingesetzter Verbund eine wasserundurchlässige Unterschicht, eine wasserdurchlässige, vorzugsweise hydrophobe, Oberschicht und eine das absorbierende, schaumförmige Polymergebilde beinhaltende Schicht, die zwischen der Unterschicht und der Oberschicht angeordnet ist. Diese das absorbierenden, schaumförmige Polymergebilde beinhaltende Schicht ist vorzugsweise ein zuvor beschriebenes Core. Die Unterschicht kann alle dem Fachmann bekannten Materialien aufweisen, wobei Polyethylen oder Polypropylen bevorzugt sind. Die Oberschicht kann gleichfalls alle dem Fachmann bekannten und geeigneten Materialien enthalten, wobei Polyester, Polyolefine, Viskose und dergleichen bevorzugt sind, die eine so poröse Schicht ergeben, die einen ausreichenden Flüssigkeitsdurchlass der Oberschicht sicherstellen. In diesem Zusammenhang wird auf die Offenbarung in US 5,061,295, US Re. 26,151, US 3,592,194, US 3,489,148 sowie US 3,860,003 verwiesen. Diese Offenbarungen werden hiermit als Referenz eingeführt und gelten somit als Teil der Offenbarung.

Die Erfindung wird nun anhand nicht limitierender Testmethoden und Beispiele näher erläutert.

**TESTMETHODEN**

BESTIMMUNG DES SCHAUMLITERGEWICHTES

Hierzu wurde eine vorgegebene Menge Zusammensetzung (A) in einem 1l Messzylinder bekannten Gewichts bis zu der 1l Marke geschäumt.

BESTIMMUNG DER *ABSORPTION UNDER LOAD* (AUL)

Zur Bestimmung der Flüssigkeitsaufnahme unter Druck (AUL) wurden 160 mg des wasserabsorbierenden, schaumförmigen Polymergebildes, das den Abmessungen des Plastikzylinders entsprach, in einen Plastikzylinder mit Siebgewebe im Boden eingewogen und mit einem definierten Gewicht belastet, welches einen Druck von 0,3 psi auf das Polymergebilde ausübt. Die Zylindereinheit wird gewogen und auf eine mit Filterpapier abgedeckte, mit 0,9 %ige NaCl-Lösung getränkte Filterplatte gestellt. Die Filterplatte liegt bis zu ihrer Oberkante in der Flüssigkeit. Überstehende Flüssigkeit ist zu vermeiden und der Flüssigkeitsstand nach 20 und 40 Minuten zu kontrollieren. Nach einer Saugzeit von 1 Stunde wir die Zylindereinheit zurückgewogen und aus der Auswaage die AUL berechnet. Die AUL ist definiert als die pro Gramm des Polymergebildes aufgenommene Gewichtsmenge an NaCl-Lösung.

**BESTIMMUNG DER ABSORPTIONSGESCHWINDIGKEIT**

Zur Bestimmung der Absorptionsgeschwindigkeit der wasserabsorbierenden, schaumförmigen Polymergebilde wird aus dem Polymergebilde eine runde Testfläche des Durchmessers 40 mm ausgest ine Petrischale des Durchmessers 60 mm werden 5 g einer mit Methylenblau angefärbten 0,9%igen Natriumchloridlösung eingewogen und eben auf eine weiße Unterlage gestellt. Die Testfläche wird aus einer Höhe von 10 mm in die mit der Lösung gefüllten Petrischale senkrecht fallengelassen. Die Zeit vom Augenblick des Auftreffens der Testfläche auf die Flüssigkeitsoberfläche bis zu dem Zeitpunkt an dem die Flüssigkeit vollständig von der Testfläche aufgenommen wurde, wird gestoppt. Es wird eine Fünffachbestimmung durchgeführt und die Aufnahmegeschwindigkeit der Gebilde in g/g/sec angegeben.

BESTIMMUNG DER MAXIMALEN ABSORPTIONSKAPAZITÄT

Zur Bestimmung der maximalen Absorptionskapazität des wasserabsorbierenden, schaumförmigen Polymergebildes wurde eine 0.9%ige NaCl-Lösung verwendet. Aus einem wasserabsorbierenden, schaumförmigen Polymergebilde wird ein Stück des Polymergebildes mit einem Gewicht von etwa 1 g ausgestanzt und einer Dicke von etwa 1 bis 5 mm, eingewogen (W1) und in einen Teebeutel eingeschweißt. Der Teebeutel wird für 30 Minuten in die Testlösung gelegt und nach einer Abtropfzeitvon 10 Minuten gewogen (W2). Einen Teebeutel ohne wasserabsorbierendes Polymer, der ebenfalls in die Testlösung gelegt und dessen Gewicht nach dem Abtropfen ebenfalls bestimmt wird (W3), lässt man als Blindwert mitlaufen. Die maximale Absorptionskapazität wurde in g/g angegeben und wie folgt berechnet:

$$CRC - Wert = \frac{W_2 - W_1 - W_3}{W_1}$$

BESTIMMUNG DER *CENTRIFUGATION RETENTION CAPACITY* (CRC)

Zur Bestimmung der Retention des schaumförmigen Gebildes wurde der Teebeuteltest durchgeführt. Als Prüflösung wurde eine 0.9%ige NaCl-Lösung verwendet. Aus einem wasserabsorbierenden, schaumförmigen Polymergebilde wird ein Stück des Polymergebildes mit einem Gewicht von etwa 1 g ausgestanzt und einer Dicke von etwa 1 bis 5 mm, eingewogen (W1) und in einen Teebeutel eingeschweißt. Der Teebeutel wird für 30 Minuten in die Testlösung gelegt und anschließend in einer Schleuder (23 cm Durchmesser, 1.400 Upm) 3 Minuten geschleudert und erneut gewogen (W2). Einen Teebeutel ohne wasserabsorbierendes Polymer, dessen Gewicht nach dem Schleudern ebenfalls bestimmt wird (W3), lässt man als Blindwert mitlaufen. Der CRC-Wert wurde in g/g angegeben und wie folgt berechnet:

$$CRC - Wert = \frac{W_2 - W_1 - W_3}{W_1}$$

BESTIMMUNG DER MITTLEREN PORENGRÖßE

Die mittlere Porengröße wurde bestimmt, in dem mittels eines Vergrößerungsglases und einem Lineal der Durchmesser von etwa 100 verschieden Poren einer relaxierten Schnittfläche des schaumförmigen Polymergebildes bestimmt und aus den so erhaltenen Messwerten der Mittelwert gebildet wurde. Diese Bestimmung wurde an 10 Schnittflächen wiederholt. Der Durchschnittswert dieser Mittelwerte entspricht der mittleren Porengröße.

EP 1 521 601 B2

BESTIMMUNG DER MITTLEREN PORENDICHTE

Die mittlere Porendichte wurde bestimmt, in dem mittels eines Vergrößerungsglases und einem Lineal in einem Areal von 1 cm × 1 cm der Oberfläche eines schaumförmigen Polymergebildes die Anzahl der in diesem Areal erkennbaren Poren ermittelt wurde. Dazu wurden die Anzahl der Poren in insgesamt etwa 10 Arealen bestimmt und aus den so erhaltenen Messwerten der Mittelwert errechnet. Dieser entspricht der mittleren Porendichte der Durchmesser von etwa 20 verschieden Poren bestimmt und aus den so erhaltenen Messwerten der Mittelwert bestimmt wurde. Dieser Mittelwert entspricht der mittleren Porengröße.

**BEISPIELE**

Beispiel 1

Herstellung einer Polyacrylsäurelösung, 3,5% Na-neutralisiert, Molekulargewicht ca. 120.000 g/mol).
Einwaagen:

| | |
|---|---|
| 225,74 g | Acrylsäure |
| 8,79 g | 50%iger wässiger Natriumhydroxidlösung |
| 677,00 g | entionsiertes Wasser |
| 0,66 g | Mercaptoethanol |
| 1,17 g | 6%iger wässrige Ascorbinsäurelösung |
| 8,34 g | 35%iges wässrige Wasserstoffperoxidlösung |
| 6,00 g | 20%ige wässrige Hydroxylaminhydrochloridlösung |

In einem Planschliffkolben werden Acrylsäure, Natriumhydroxidlösung und 477,00 g entionisierten Wasser gegeben. Es wird eine Stunde Stickstoff durch die Lösung geleitet und auf 30 °C erwärmt. Unter Rühren werden Mercaptoethanol, Ascorbinsäurelösung und 1,45 g Wasserstoffperoxidlösung zugegeben, wonach die Temperatur der Lösung auf ca. 85°C steigt. Man lässt 30 min bei einer Badtemperatur von 80°C rühren und gibt dann 6,00 g Hydroxylaminhydrochloridlösung und 6,89 g Wasserstoffperoxidlösung hinzu. Das Heizbad wird ausgeschaltet, und 200,00 g entionisiertes Wasser zugegeben, so dass eine wässrige 25%ige Polymerlösung entsteht.

**Beispiel 2**

Herstellung eines Acrylsäure-Hydroxyethylmethacrylat-Copolymers, 3,5% Na-neutralisiert, Molekulargewicht ca. 145.000 g/mol.
Einwaagen:

| | |
|---|---|
| 217,72 g | Acrylsäure |
| 8,03 g | Hydroxyethylmethacrylat |
| 8,48 g | 50%iger wässiger Natriumhydroxidlösung |
| 677,00 g | entionsiertes Wasser |
| 0,66 g | Mercaptoethanol |
| 1,17 g | 6%iger wässrige Ascorbinsäurelösung |
| 8,34 g | 35%iges wässrige Wasserstoffperoxidlösung |
| 6,00 g | 20%ige wässrige Hydroxylaminhydrochloridlösung |

In einem Planschliffkolben werden Acrylsäure, Hydroxyethlymethacrylat und Natriumhydroxidlösung in 477,00 g entionisierten Wasser gegeben. Es wird eine Stunde Stickstoff durch die Lösung geleitet und auf 30°C erwärmt. Unter Rühren werden Mercaptoethanol, Ascorbinsäurelösung und 1,45 g Wasserstoffperoxidlösung zugegeben, wonach die Temperatur der Lösung auf ca. 100°C steigt. Man lässt 2 Stunden bei einer Badtemperatur von 80°C rühren und gibt dann 6,00 g Hydroxylaminhydrochloridlösung und 6,89 g Wasserstoffperoxidlösung hinzu und lässt eine weitere Stunde bei einer Badtemperatur von 80°C rühren. Das Heizbad wird ausgeschaltet, und 200,00 g entionisiertes Wasser zugegeben, so dass eine wässrige 25%ige Polymerlösung entsteht.

Beispiel 3

Herstellung eines Acrylsäure- Butylacrylat-Copolymers, 3,5% Na-neutralisiert, Molekulargewicht ca. 420.000 g/mol.

Einwaagen:

| | |
|---|---|
| 213,97 g | Acrylsäure |
| 11,77 g | Butylacrylat |
| 8,34 g | 50%iger wässiger Natriumhydroxidlösung |
| 677,00 g | entionsiertes Wasser |
| 0,44 g | Mercaptoethanol |
| 1,17 g | 6%iger wässrige Ascorbinsäurelösung |
| 7,15 g | 35%iges wässrige Wasserstoffperoxidlösung |
| 6,00 g | 20%ige wässrige Hydroxylaminhydrochloridlösung |

In einem Planschliffkolben werden Acrylsäure und Natriumhydroxidlösung in 477,00 g entionisierten Wasser gegeben. Es wird eine Stunde Stickstoff durch die Lösung geleitet und auf 30 °C erwärmt. Unter Rühren werden Mercaptoethanol, Ascorbinsäurelösung und 1,24 g Wasserstoffperoxidlösung zugegeben, sowie zeitgleich innerhalb von drei Minuten Butylacrylat eingetropft. Die Temperatur der Lösung steigt dabei rasch auf ca. 96°C. Man lässt 2 Stunden bei einer Badtemperatur von 80°C rühren und gibt dann 6,00 g Hydroxylaminhydrochloridlösung und 5,91 g Wasserstoffperoxidlösung hinzu und lässt eine weitere Stunde bei einer Badtemperatur von 80°C rühren. Das Heizbad wird ausgeschaltet, und 200,00 g entionisiertes Wasser zugegeben, so dass eine wässrige, hochviskose 25%ige Polymerlösung entsteht.

Beispiel 4

Erfindungsgemäße Herstellung eines wasserabsorbierenden, schaumförmigen Polymergebildes.

| | |
|---|---|
| 33,35 g | Polymer aus Beispiel 1 |
| 0,4 g | Stokal SR (Gebrüder Langefeld, Krefeld) |
| 0,5 g | Kaliumstearat |
| 0,5 g | Glucopon 225 CS UP (Henkel KGaA, Düsseldorf) |
| 12,18 g | 9,71 %ige Natronlauge |
| 16,71 g | 15,34%ige Kalilauge |
| 0,15 g | Denacol Ex 810 (Nagase Kaseikogyo, Japan) |
| 8,22 g | Citronensäuremonohydrat |
| 4,0 g | Ethylencarbonat |

werden zusammengegeben und mittels eines Krupps 3 Mix-Gerätes für drei Minuten aufgeschlagen. Der entstandene Schaum wird auf einer Fläche von 0,1 m$^2$ und auf eine Höhe von 0,2 cm ausgestrichen und 15 min bei 220 °C im Umlufttrockenschrank erhitzt. Das entstandene Gebilde besitzt folgende Kenndaten:

Tabelle 1

| Maximale Absorptionskapazität [g/g] | CRC [g/g] | AUL (0,3 psi) [g/g] | Absorptionsgeschwindigkeit [g/g/sec] |
|---|---|---|---|
| 95,1 | 31,2 | 25,6 | 7,85 |

Beispiel 5

Erfindungsgemäße Herstellung eines wasserabsorbierenden, schaumförmigen Polymergebildes.

Analog zu Beispiel 4 werden 33,35 g Copolymer zu wasserabsorbierenden, schaumförmigen Polymergebilden verarbeitet. Die Herstellung des Copolymers erfolgt analog zu Beispiel 1, wobei in dem in der Tabelle 2 angegebene molaren Verhältnis der entsprechende Anteil an Acrylsäure durch Comonomer ausgetauscht worden ist.

Tabelle 2

| Copolymer der Monomerkombination (mol%-Anteil) | Maximale Absorptionskapazität (g/g) | CRC (g/g) | AUL (0.3 psi) (g/g) | Absorptionsgeschwindigkeit (g/g/sec) |
|---|---|---|---|---|
| AS/BA(98/2) | 46,2 | 15,2 | 11,3 | 6,9 |
| ASBA (99,5/0,5) | 64,1 | 17,5 | 13,1 | 6,6 |
| ASBA/HEMA (99/0,5/0,5) | 45,8 | 15,8 | 11,7 | 5,2 |
| AS/PEG-MAE (98/2) | 43,5 | 14,5 | 12,4 | 7,0 |
| AS/PEG-MAE (96/4) | 25,5 | 8,1 | 9,5 | 4,5 |
| AS/PEG-MAE (99/1) | 55,8 | 16,6 | 11,9 | 7,5 |
| ASBA/PEG-MAE (96/2/2) | 50,3 | 16,0 | 11,7 | 5,1 |
| ASBA/PEG-MAE (97/2/1) | 46,2 | 15,1 | 12,5 | 6,3 |
| ASBA/PEG-MAE (97/1/2) | 49,1 | 15,1 | 12,2 | 7,1 |
| ASBA/PEG-MAE (98/1/1) | 55,8 | 17,7 | 12,2 | 6,4 |
| AS=Acrylsäure BA=Butylacrylat HEMA=Hydroxyethylmethacrylsäure PEG-MAE=Polyethylenglykolmonoallylether | | | | |

Beispiel 6

Erfindungsgemäße Herstellung eines wasserabsorbierenden, schaumförmigen Polymergebildes. Analog zu Beispiel 4 werden 33,35 g Copolymer zu wasserabsorbierenden, schaumförmigen Polymergebilden verarbeitet. Die Herstellung des Copolymers erfolgt analog zu Beispiel 2, wobei entstehend dem in der Tabelle 3 angegebene molare Verhältnis eingestellt worden ist.

Tabelle 3

| Copolymer der Monomerkombination (mol%-Anteil) | Maximale Absorptionskapazität (g/g) | CRC (g/g) | AUL (0.3 psi) (g/g) | Absorptionsgeschwindigkeit (g/g/sec) |
|---|---|---|---|---|
| ASBA (98/2) | 50,5 | 15,3 | 12,5 | 2,3 |
| ASBA (96/4) | 37,9 | 14,3 | 13,2 | 3,1 |
| AS/BA(99/1) | 59,5 | 18,4 | 11,8 | 9,2 |
| AS/BA (99,5/0,5) | 54,4 | 18,8 | 11,8 | 9,5 |
| AS/HEMA (98/2) | 41,2 | 14,6 | 13,0 | 9,1 |
| AS/BAMEMA (98/1/1) | 43,1 | 13,5 | 13,0 | 7,4 |
| AS/PEG-MAE (98/2) | 44,3 | 13,4 | 12,0 | 6,2 |
| AS/BA/PEG-MAE (96/2/2) | 37,5 | 12,4 | 12,0 | 4,4 |
| ASBA (97/3) | 39,2 | 16,0 | 14,4 | 1,6 |
| ASBA/PEG-MAE (96,5/3/0,5) | 49,0 | 17,4 | 12,6 | 4,2 |
| ASBA/PEG-MAE (96/3/1) | 45,4 | 13,2 | 12,7 | 3,7 |
| AS=Acrylsäure BA=Butylacrylat HEMA=Hydroxyethylmethacrylsäure PEG-MAE=Polyethylenglykolmonoallylether | | | | |

Beispiel 7

Herstellung eines wasserabsorbierenden, schaumförmigen Polymergebildes. Zwei Polymere, hergestellt analog zu Beispiel 2, werden in dem in Tabelle 4 angegebenen Verhältnis gemischt und analog zu Beispiel 4 verarbeitet.

Tabelle 4

| Copolymer der Monomerkombination (mol%-Anteil) | Maximale Absorptionskapazität (g/g) | CRC (g/g) | AUL (0.3 psi) (g/g) | Absorptionsgeschwindigkeit (g/g/sec) |
|---|---|---|---|---|
| 10% AS/PEG-MAE (98/2) 90% AS/BA (98/2) | 49,0 | 18,2 | 15,2 | 2,6 |
| 25% AS/PEG-MAE (98/2) 75% AS/BA (98/2) | 54,0 | 17,7 | 15,0 | 3,8 |
| 50% AS/PEG-MAE (98/2) 50% ASBA (98/2) | 51,0 | 17,8 | 14,7 | 5,8 |
| 10% AS/PEG-MAE (98/2) 90% ASBA (96/4) | 43,2 | 16,4 | 14,1 | 3,2 |
| 25% AS/PEG-MAE (98/2) 75% ASBA (96/4) | 51,9 | 16,7 | 12,3 | 3,4 |
| 50% AS/PEG-MAE (98/2) 50% ASBA (96/4) | 50,6 | 15,4 | 13,5 | 3,8 |
| AS=Acrylsäure BA=Butylacrylat HEMA=Hydroxyethylmethacrylsäure PEG-MAE=Polyethylenglykolmonoallylether | | | | |

Beispiel 8

Erfindungsgemäße Herstellung eines wasserabsorbierenden, schaumförmigen Polymergebildes mit alternativen Vernetzern. Das Polymer wird analog zu Beispiel 2 hergestellt, wobei Butylacrylat nach Anspringen der Polymerisation innerhalb von 3 Minuten zugetropft wird. Die Herstellung der wasserabsorbierenden, schaumförmigen Polymergebilde erfolgt analog zu Beispiel 4. Variiert ist Denacol Ex 810 gegen die in der Tabelle 5 angegebenen Vernetzer.

Tabelle 5

| Vernetzer | Vernetzermenge [g/g] | maximale Absorptionkapazität [g/g] | CRC [g/g] | AUL (0,3 psi) [g/g] |
|---|---|---|---|---|
| Pentaerythrit | 0,15 | 32,8 | 15,8 | 13,3 |
| Glycerin | 0,20 | 34,2 | 15,9 | 13,1 |
| Sorbit | 0,15 | 41,3 | 16,7 | 14,5 |
| Glykol | 0,15 | 38,3 | 17,1 | 12,9 |
| PEG 600 | 0,30 | 47,6 | 18,4 | 15,6 |
| PEG 300 | 0,30 | 31,0 | 14,8 | 15,4 |
| Schleimsäure | 0,15 | 41,8 | 18,22 | 12,5 |
| Denacol Ex 810 | 0,15 | 39,2 | 16,0 | 14,4 |

Erfindungsgemäße Herstellung eines wasserabsorbierenden, schaumförmigen Polymergebildes. Analog zu Beispiel 4 werden 33,35 g Copolymer (97 mol Acrylsäure : 3 mol Butylacrylat) zu einem wasserabsorbierenden, schaumförmigen Polymergebilde verarbeitet.

Tabelle 6

| maximale Absorptionkapazität [g/g] | CRC [g/g] | AUL (0,3 psi) [g/g] | Absorptionsgeschwindigkeit [g/g/sec] |
|---|---|---|---|
| 35,3 | 11,4 | 17,5 | 2,09 |

Beispiel 10

Die erfindungsgemäße Herstellung eines Polymergebildes aus einem Acrylsäure-Butylacrylat-Copolymer (97 mol: 3 mol) hergestellt nach Beispiel 3, wobei jedoch bei Menge an Mercaptoethanol auf 0,22 g reduziert wird. Analog zu Beispiel 4 werden 33,35 g Copolymer zu einem wasserabsorbierenden, schaumförmigen Polymergebilde verarbeitet.

Tabelle 7

| maximale Absorptionkapazität [g/g] | CRC [g/g] | AUL (0,3 psi) [g/g] | Absorptionsgeschwindigkeit [g/g/sec] |
|---|---|---|---|
| 27,5 | 7,7 | 16,4 | 2,04 |

Beispiel 11

Die erfindungsgemäße Herstellung eines Polymergebildes aus einem Acrylsäure-polymer hergestellt nach Beispiel 1, wobei jedoch bei Menge an Mercaptoethanol auf 0,44 g reduziert wird. Analog zu Beispiel 4 werden 33,35 g Copolymer zu einem wasserabsorbierenden, schaumförmigen Polymergebilde verarbeitet.

Tabelle 8

| maximale Absorptionkapazität [g/g] | CRC [g/g] | AUL (0,3 psi) [g/g] | Absorptionsgeschwindigkeit [g/g/sec] |
|---|---|---|---|
| 44,0 | 14,3 | 15,4 | 6,94 |

Beispiel 12

Die erfindungsgemäße Herstellung eines Polymergebildes aus einem Acrylsäure-polymer hergestellt nach Beispiel 1, wobei jedoch bei Menge an Mercaptoethanol auf 0,22g reduziert wird. Analog zu Beispiel 4 werden 33,35 g Copolymer zu einem wasserabsorbierenden, schaumförmigen Polymergebilde verarbeitet.

Tabelle 9

| maximale Absorptionkapazität [g/g] | CRC [g/g] | AUL (0,3 psi) [g/g] | Absorptiongeschwindigkeit [g/g/sec] |
|---|---|---|---|
| 32,1 | 9,6 | 17,3 | 5,66 |

Beispiel 13

Erfindungsgemäße Herstellung eines wasserabsorbierenden, schaumförmigen Polymergebildes aus einem Acrylsäure-Butylacrylat-Copolymer (97 mol :3 mol) mit alternativen Vernetzern. Das Polymer aus Beispiel 3 wird verwendet. Die Herstellung der Gebilde erfolgt analog zu Beispiel 4. Ausgetauscht ist Denacol Ex810 gegen die in der Tabelle angegebenen Vernetzer.

Tabelle 10

| Vernetzer | Vernetzermenge [g/g] | maximale Absorptionkapazität [g/g] | CRC [g/g] | AUL (0,3 psi) [g/g] |
|---|---|---|---|---|
| Sorbit | 0,25 | 48,6 | 15,4 | 20,2 |
| PEG 600 | 0,25 | 51,2 | 15,4 | 21,7 |
| Schleimsäure | 0,25 | 66,3 | 19,0 | 20,4 |
| Hydroxyethylcellulose | 0,25 | 54,2 | 15,7 | 20,1 |
| Kymene ULX | 0,17g | 81,6 | 24,5 | 17,2 |
| Kymene ULX | 0,25g | 58,2 | 18,8 | 19,3 |

Beispiel 14

Erfindungsgemäße Herstellung eines wasserabsorbierenden, schaumförmigen Polymergebildes aus einem Acrylsäure-Polymer, hergestellt nach Beispiel 1, wobei jedoch bei Menge an Mercaptoethanol auf 0,44 g reduziert wird. Die Herstellung der Gebilde erfolgt analog zu Beispiel 4. Ausgetauscht ist Denacol Ex 810 gegen die in der Tabelle angegebenen Vernetzer.

Tabelle 11

| Vernetzer | Vernetzermenge [g/g] | maximale Absorptionkapazität [g/g] | CRC [g/g] | AUL (0,3 psi) [g/g] |
|---|---|---|---|---|
| Sorbit | 0,25 | 58,5 | 20,9 | 15,0 |
| Schleimsäure | 0,25 | 77,3 | 28,6 | 14,1 |
| Schleimsäure | 0,42 | 60,3 | 17,0 | 19,2 |
| Pentaerythrit | 0,25 | 67,5 | 20,1 | 15,5 |
| Trimethylolpropan | 0,25 | 64,4 | 20,9 | 16,6 |
| Hydroxyethylcellulose | 0,25 | 68,4 | 22,7 | 14,4 |
| Kymene ULX | 0,17g | 79,9 | 21,6 | 19,5 |
| Kymene ULX | 0,25g | 73,8 | 25,1 | 17,1 |

Beispiel 15

Erfindungsgemäße Herstellung eines wasserabsorbierenden, schaumförmigen Polymergebildes aus Acrylsäure-Polymeren mit unterschiedlichen Tensidzusätzen.

| | |
|---|---|
| 33,35 g | Polymer aus Beispiel 1 |
| 0,4 g | Stokal SR |
| 0,5 g | Tensid, wie in Tabelle angegeben |
| 33,42 g | 15,34%ige Kalilauge |
| 0,15 g | Denacol Ex 810 |
| 8,22 g | Citronensäuremonohydrat |
| 2,0 g | Ethylencarbonat |

werden zusammengegeben und mittels eines Krupps 3 Mix-Gerätes für drei Minuten aufgeschlagen. Der entstandene Schaum wird auf einer Fläche von $0,1.m^2$ und auf eine Höhe von 0,2 cm ausgestrichen und 15 min bei 220 °C im Umlufttrockenschrank erhitzt.

Das entstandene Polymergebilde besitzt folgende Kenndaten:

Tabelle 12

| Tensid | Maximale Absorptionkapazität [g/g] | CRC [g/g] | AUL (0,3 psi) [g/g] |
|---|---|---|---|
| Glucopon 225 CS UP | 67,9 | 20,9 | 14,9 |
| Glucopon EC 650 | 67,3 | 21,4 | 14,6 |
| Rewoteric AM 2C NM | 58,1 | 21,9 | 13,6 |
| Rewoteric AM R 40 | 61,9 | 20,0 | 16,1 |
| Rewoteric AM KSF 40 | 73,7 | 20,3 | 16,7 |
| Elfan NS 242 A | 64,1 | 19,6 | 14,7 |
| Kaliumstearat | 80,2 | 23,9 | 14,8 |
| Cocoamidopropylbetain | 68,1 | 19,7 | 14,3 |

Beispiel 16

Erfindungsgemäße Herstellung eines wasserabsorbierenden, schaumförmigen Polymergebildes aus Acrylsäure-Polymer mit einer wasserundurchlässigen Rückseite.

| | |
|---|---|
| 33,35 g | Polymer aus Beispiel 1 |
| 0,4 g | Stokal SR |
| 0,5 g | Kaliumstearat |
| 0,5 g | Glucopon 225 CS UP (Henkel, Düsseldorf) |
| 12,18 g | 9,71%ige Natronlauge |
| 16,71 g | 15,34%ige Kalilauge |
| 0,15 g | Denacol Ex 810 |
| 8,22 g | Citronensäuremonohydrat |
| 4,0 g | Ethylencarbonat |
| 1,00 | Estekoll HL 50/200 |
| 1,00 | Sarpifan PA 308 A (Stockhausen GmbH & Co. KG, Krefeld) |

werden zusammengegeben und mittels eines Krupps 3 Mix-Gerätes für drei Minuten aufgeschlagen. Der entstandene. Schaum wird auf einer Fläche von 0,1 m2 und auf eine Höhe von 0,2 cm ausgestrichen und 15 min bei 220 °C im Umlufttrockenschrank erhitzt. Das erhaltene Polymergebilde wird auf eine Polyethylenfolie (PE-Folie) gelegt und zwischen silikonisierten Papierstreifen 10 min lang mit einem Bügeleisen bis zur vollständigen Anhaftung des Gebildes an die Folie gebügelt.

Tabelle 13

| | maximale Absorptionkapazität [g/g] | CRC [g/g] | AUL (0,3 psi) [g/g] |
|---|---|---|---|
| Vor dem Kalandern auf PE-Folie | 78,1 | 26,7 | 22,6 |
| Nach dem Kalandern auf PE-Folie | 69,0 | 20,0 | 16,1 |

Beispiel 17

100 g eines nach Beispiel 4 hergestellten Schaums wurden gleichmäßig mit einer Lösung aus 15 g Wasser und 1,5 g Ethylencarbonat besprüht. Nach dem sich die Ethylencarbonat -Lösung in dem Schaum über eine Stunde gleichmäßig verteilt hatte, wurde der mit Ethylencarbonat beladene Schaum über 60 Minuten bei 180°C nachvemetzt und getrocknet. Es wurde ein nachvernetzter Schaum mit Eigenschaften gemäß Tabelle 14 erhalten.

Tabelle 14

| Maximale Absorptionskapazität [g/g] | CRC [g/g] | AUL (0,3 psi) [g/g] | Absorptionsgeschwindigkeit [g/g/sec] |
|---|---|---|---|
| 95,1. | 28,2 | 29,5 | 9,1 |

**Patentansprüche**

1.  Ein Verfahren zur Herstellung von wasserabsorbierenden, schaumförmigen Polymergebilden, wobei eine wässrige Zusammensetzung (A) beinhaltend

    (A1) Wasser,
    (A2) ein oder mehrere Polymere, welche mindestens auf

    ($\alpha$1) 55-100 Gew.-% einem polymerisierten, monoethylenisch ungesättigten, säuregruppenhaltigen Monomeren oder dessen Salz sowie auf
    ($\alpha$2) 0-45 Gew.-% einem polymerisierten, monoethylenisch ungesättigten, mit ($\alpha$1) copolymerisierbaren Monomeren basiert,
    wobei die Summe der Gewichtsmengen ($\alpha$1) und ($\alpha$2) 100 Gew.-% beträgt und wobei mindestens 31,5 Gew.-% der Monomeren, bezogen auf das Gesamtgewicht der Monomeren ($\alpha$1) und ($\alpha$2), Acrylsäure oder Salze der Acrylsäure sind,

(A3) einen oder mehrere Vernetzer,
(A4) ein oder mehrere Blähmittel,
(A5) ein oder mehrere Tenside,
(A6) sowie gegebenenfalls weitere Hilfsstoffe
aufgeschäumt und die geschäumte, wässrige Zusammensetzung anschließend bei einer Temperatur in einem Bereich von 50 bis 300°C erhitzt wird, so dass die Polymere (A2) zumindest teilweise vernetzt und der Gehalt an Wasser (A1) auf höchstens 15 Gew.-%, bezogen auf das Gesamtgewicht des entstehenden, schaumförmigen Polymergebildes, eingestellt wird.

2. Verfahren nach Anspruch 1, wobei das Polymer (A2) ein Zahlenmittel des Molekulargewichtes von mindestens 10.000 g/mol aufweist.

3. Verfahren nach Anspruch 1 oder 2, wobei die geschäumte Zusammensetzung ein Schaumlitergewicht von 10 bis 1.000 g/l aufweist.

4. Verfahren nach einem oder mehreren der Ansprüche 1 bis 3, wobei die Oberfläche des absorbierenden, schaumförmigen Polymergebildes in einem weiteren Verfahrensschritt geglättet wird.

5. Verfahren zur Herstellung eines Verbundes umfassend ein wasserabsorbierendes, schaumförmiges Polymergebildes erhältlich nach einem Verfahren gemäß Anspruch 1 bis 4 und ein Substrat, wobei eine geschäumte Zusammensetzung, wie in Anspruch 1 bis 3 definiert, mit mindestens einem Teil der Oberfläche eines Substrates in Kontakt gebracht wird und das mit der geschäumten Zusammensetzung in Kontakt gebrachte Substrat anschließend bei einer Temperatur in einem Bereich von 50 bis 300°C erhitzt wird, so dass die Polymere (A2) zumindest teilweise vernetzt, der Gehalt an Wasser (A1) auf höchstens 15 Gew.-%, bezogen auf das Gesamtgewicht des entstehenden, schaumförmigen Polymergebildes, eingestellt und das entstehende, schaumförmige Polymergebilde auf zumindest einem Teil der Substratoberfläche immobilisiert wird.

6. Verfahren nach Anspruch 5, wobei das Substrat eine Folie aus Polymeren, wie beispielsweise aus Polyethylen, Polypropylen oder Polyamid, ein Metall, ein Vlies, ein Fluff, ein Tissue, eine Gewebe, eine natürliche oder synthetische Faser oder ein anderer Schaum ist.

7. Verfahren nach Anspruch 5 oder 6, wobei bei dem Aufbringen der geschäumten, wässrigen Zusammensetzung auf das Substrat Schablonen verwendet werden.

**Claims**

1. A process for the preparation of water-absorbing, foamed polymer structures, wherein an aqueous composition (A) comprising

(A1) water,
(A2) one or more polymers which are based at least on

($\alpha$1) 55 - 100 wt.% of a polymerized, monoethylenically unsaturated, acid group-containing monomer or a salt thereof and on
($\alpha$2) 0 - 45 wt.% of a polymerized, monoethylenically unsaturated monomer which can be copolymerized with ($\alpha$1),
wherein the sum of the amounts by weight ($\alpha$1) and ($\alpha$2) is 100 wt.% and wherein at least 31.5 wt.% of the monomers, based on the total weight of the monomers ($\alpha$1) and ($\alpha$2), is acrylic acid or salts of acrylic acid,

(A3) one or more crosslinking agents,
(A4) one or more blowing agents,
(A5) one or more surfactants,
(A6) and optionally further auxiliaries
is foamed and the foamed aqueous composition is then heated at a temperature in a range of from 50 to 300 °C, so that the polymers (A2) are at least partly crosslinked and the content of water (A1) is adjusted to at most 15 wt.%, based on the total weight of the foamed polymer structure formed.

**2.** Process according to claim 1, wherein the polymer (A2) has a number-average molecular weight of at least 10,000 g/mol.

**3.** Process according to claim 1 or 2, wherein the foamed composition has a foam density of from 10 to 1,000 g/l.

**4.** Process according to one or more of claims 1 to 3, wherein the surface of the absorbent foamed polymer structure is smoothed in a further process step.

**5.** A process for the production of a composite comprising a water-absorbing, foamed polymer structure obtainable the process according to claim 1 to 4 and a substrate, wherein a foamed composition as defined in claim 1 to 3 is brought into contact with at least a part of the surface of a substrate and the substrate brought into contact with the foamed composition is then heated at a temperature in a range of from 50 to 300 °C, so that the polymers (A2) are at least partly crosslinked, the content of water (A1) is adjusted to at most 15 wt.%, based on the total weight of the foamed polymer structure formed, and the foamed polymer structure formed is immobilized on at least a part of the substrate surface.

**6.** Process according to claim 5, wherein the substrate is a film of polymers, such as, for example, of polyethylene, polypropylene or polyamide, a metal, a nonwoven, a fluff, a tissue, a woven fabric, a natural or synthetic fibre or another foam.

**7.** Process according to claim 5 or 6, wherein templates are used during application of the foamed aqueous composition to the substrate.


**Revendications**

**1.** Procédé pour la préparation d'entités polymères en forme de mousse absorbant l'eau, dans lesquelles une composition aqueuse (A) contenant

(A1) de l'eau,
(A2) un ou plusieurs polymères, lesquels sont basés au minimum sur

($\alpha$1) 55 à 100% en poids d'un monomère polymérisé, monoéthyléniquement insaturé, contenant des groupes acides ou un sel de ce dernier, de même que sur
($\alpha$2) 0 à 45% en poids d'un monomère polymérisé, monoéthyléniquement insaturé, copolymérisable avec ($\alpha$1),

la somme des quantités en poids ($\alpha$1) et ($\alpha$2) étant égale à 100% en poids et au moins 31,5% en poids des monomères, par rapport au poids total des monomères ($\alpha$1) et ($\alpha$2), est constitué d'acide acrylique ou de sels de l'acide acrylique,
(A3) un ou plusieurs réticulants,
(A4) un ou plusieurs gonflants,
(A5) un ou plusieurs tensides,
(A6) ainsi que, le cas échéant, d'autres consommables secondaires, est moussée et la composition aqueuse moussée est réchauffée ensuite à une température se situant dans un domaine de 50 à 300°C, de façon à ce que les polymères (A2) soient au moins partiellement réticulés et la teneur en eau (A1) soit ajustée à 15% maximum, par rapport au poids total de l'entité polymère en forme de mousse formée.

**2.** Procédé suivant la revendication 1, dans lequel le polymère (A2) présente une moyenne numérique du poids moléculaire d'au moins 10.000 g/mole.

**3.** Procédé suivant la revendication 1 ou 2, dans lequel la composition en forme de mousse présente un poids par litre de mousse de 10 à 1.000 g/l.

**4.** Procédé suivant une ou plusieurs des revendications 1 à 3, dans lequel la surface de l'entité polymère absorbant en forme de mousse est lissée au cours d'une étape de procédé ultérieure.

5. Procédé pour la préparation d'un composé comprenant une entité polymère absorbant l'eau en forme de mousse qui peut être obtenue suivant un procédé conformément aux revendications 1 à 4 et un substrat, dans lequel une composition en forme de mousse, telle que définie dans les revendications 1 à 3, est mise en contact avec au moins une partie de la surface d'un substrat et le substrat mis en contact avec la composition en forme de mousse est ensuite chauffé à une température se situant dans un domaine de 50 à 300°C, de manière telle que les polymères (A2) soient au moins partiellement réticulés, que la teneur en eau (A1) soit ajustée à un maximum de 15% en poids, par rapport au poids total de l'entité polymère en forme de mousse obtenue et que l'entité polymère en forme de mousse obtenue soit au moins immobilisée sur une partie de la surface du substrat.

6. Procédé suivant la revendication 5, dans lequel le substrat est une feuille constituée de polymères, comme par exemple un polyéthylène, polypropylène ou un polyamide, un métal, un voile, un fluff, un tissue, un tissu, une fibre naturelle ou synthétique ou une autre mousse.

7. Procédé suivant la revendication 5 ou 6, des gabarits étant utilisés pour l'application de la composition aqueuse mousseuse sur le substrat.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0427219 A
- WO 9621680 A
- WO 9621681 A
- WO 9422502 A
- WO 9717397 A
- US 4394930 A
- WO 8809801 A
- US 5061295 A
- US RE26151 E
- US 3592194 A
- US 3489148 A
- US 3860003 A

**In der Beschreibung aufgeführte Nicht-Patentliteratur**

- **R. Y. LOCHHEAD ; W. R. FRON.** *Cosmetics & Toiletries,* Mai 1993, vol. 108, 95-135
- **M. T. CLARKE.** Rheological Additivs. D. Laba
- Rheological Properties of Cosmetics and Toiletries. Cosmetic Science and Technology Series. Marcel Dekker Inc, 1993, vol. 13